# EUROPEAN PATENT APPLICATION

(11) **EP 4 495 606 A1**
(43) Date of publication of application: **22.01.2025**
(21) Application number: 23852855.8
(22) Date of filing: 01.08.2023
(51) Int. Cl.: G01N 35/10, G01N 21/84, C12M 1/26, C12M 1/36, G01N 35/00

(54) **AUTOMATIC CELL DISPENSING DEVICE, LIQUID HANDLING DEVICE INCLUDING SAME, AND AUTOMATIC CELL DISPENSING METHOD**

(30) Priority: 10.08.2022 KR 20220100077
(71) Applicant: Able Labs Inc., Incheon 21984 (KR)
(72) Inventor: SHIN, Sang, Incheon 21982 (KR); KO, Nam Il, Incheon 21997 (KR); ROH, Hyung Rae, Incheon 22762 (KR); PARK, Sang Young, Siheung-si Gyeonggi-do 14992 (KR); PARK, Jae Seong, Incheon 22021 (KR)
(74) Representative: Schön, Christoph
(86) International application number: PCT/KR2023/011251
(87) International publication number: WO 2024/034975

(57) **Abstract**

An automatic liquid handling device comprises: a deck, located at the bottom, for accommodating one or more racks; a pipetting device, placed above the deck, capable of three-dimensional movement by an actuator, for aspirating and dispensing samples by coupling pipette tips to a probe; a tip optical system, mounted on the deck, for imaging a cell mixture aspirated into the pipette tip from a sample container at the counting position; a cooling module that holds a sample container accommodating a cell mixture containing cells or cell clusters, to keep the cell mixture cool; and a control module for calculating the number of cells based on the taken image of the cell mixture aspirated into the pipette tip and controlling the operation of the pipetting device according to a pre-programmed method, wherein the control module calculates the number of cells on the basis of the taken image of the pipette tip.

## Description

### [Technical Field]

The present invention relates to an automatic cell dispensing device and method, and more particularly, to an automatic cell dispensing device and method using an optical counter, and a liquid handling system including the automatic cell dispensing device.

### [Background Art]

The focus of the pharmaceutical industry is shifting from generic medicines to biomedicines. Cells collected from animals or humans are cultured in media under laboratory conditions (in vitro) and used for various purposes for conducting various reaction experiments or growing the cells into desired tissues. Recently, three-dimensional (3D) cell culture, which is particularly similar to culture in the in vivo environment, has been in the spotlight.

In various experiments using cell suspensions, for example, cell mixtures obtained by mixing cells and matrigels (Matrigel^{®} Matrix by Corning, a natural ECM-based hydrogel), it is necessary to dispense a large number of cells onto a plate for cell culture or reaction experiments. To ensure reliability and reproducibility of experimental results, each dispensing should be performed uniformly. By uniformly dispensing single cells or cell aggregates (hereinafter referred to as "cells"), when various experiments such as an experiment of culturing a large number of dispensed cells/cell groups, an experiment of adding medicines, or a protein overexpression experiment are performed, uniform and reproducible experimental results may be obtained. Therefore, dispensing cells such that the number of cells included in each dispensed cell mixture is uniform is an important task in the art.

Typically, cell aggregates or cells such as organoids or spheroids contained in a sample container (tube) are suctioned into a pipette tip and then dispensed onto a plate and thus form a dome array or are each dispensed into a plurality of wells of a well plate. Conventionally, cell dispensing was performed based on dilution. Dilution-based dispensing is based on the assumption that cells (or cell aggregates or cells) are entirely distributed uniformly in a cell suspension contained in a sample tube. That is, under the assumption that the same number of cells is included in each cell suspension with the same volume, the dilution-based dispensing is a method of manually or automatically suctioning the cell suspensions with the same volume into a pipette tip and dispensing the cell suspensions onto a plate as a plurality of domes or the like.

However, this method has limitations because there may be errors according to experimenter' skills and various environmental conditions. For example, when cells settle to a bottom of a sample tube over time, more cells may be included in the same amount (volume) of suspension over time, or conversely, an amount of cells may decrease over time. In addition, the distribution of cells in a suspension varies according to environmental conditions such as temperature, and thus the same amount of cell suspension cannot guarantee the same number of cells. Therefore, there is a need to overcome the limitations of this dilution-based dispensing, and there is a need for more accurate and uniform cell dispensing automation.

(Patent Document 1) KR 10-1858056 B

### [Disclosure]

### [Technical Problem]

The present invention is directed to solving the problems of dilution-based dispensing and providing an automatic cell dispensing method and device for uniformly dispensing cells.

The present invention is also directed to providing a method of uniformly dispensing cells using an automatic liquid handling system having a modularized and compact configuration.

### [Technical Solution]

In order to solve the above object, an automatic cell dispensing device according to one aspect of the present invention is for automatically and uniformly dispensing a cell mixture and dispenses a cell mixture by performing automatic control such that the number of cells included in the dispensed cell mixture is uniform.

The automatic cell dispensing device includes a pipette device which is movable along three axes by an actuator and in which a pipette tip is coupled to a probe to suction and dispense a cell mixture, and a tip vision module configured to capture an image of the pipette tip in which the cell mixture is suctioned from a sample container by the pipette device at a counting position. It is preferable that the number of cells is calculated based on the captured image of the cell mixture suctioned into the pipette tip.

The tip vision module may include an imaging module and a lighting module, the imaging module may include a first optical system including one or more lenses and a first camera, and the lighting module may include a first lighting unit having an annular shape.

The first optical system and the first camera may be disposed such that the first camera captures an image of the counting position at an annular center of an upper end portion of the first lighting unit. The sample container may be seated to be coaxially positioned below the first lighting unit along an annular central axis thereof, and in a state in which the cell mixture from the sample container is suctioned into the pipette tip, the pipette device may be controlled to be vertically lifted and moved to the counting position.

A cooling module on which the sample container is seated may be disposed below the counting position. And the cooling module may include a thermoelectric element, a heat sink coupled to and in close contact with a lower portion of the thermoelectric element, one or more fans, a holder positioned on the thermoelectric element, and a cooler case.

The holder may have an upper end that is open such that the sample container is inserted from above and seated, and a lower end that is directly coupled to and in close contact with an upper end of the thermoelectric element or coupled through a heat transfer unit. And the cooler case may accommodate the thermoelectric element, the heat sink, and the one or more fans therein.

When a certain amount of a sample is suctioned into the pipette tip from the sample container by the pipette device and then is moved upward and positioned at the counting position, an image of a certain area of a lower portion of the pipette tip may be captured from a side by the tip vision module.

The automatic cell dispensing device may further include an information processing device which is part of the automatic cell dispensing device or a separate external device, and the information processing device may include a processor, a memory, and a communication module and may be for analyzing the captured image and calculating the number of cells in a certain area of the pipette tip. A process of capturing an image and calculating the number of cells through image analysis may be repeated one or more times until the calculated number of cells reaches a certain number, and when the calculated number of cells reaches the certain number, the pipette device may dispense a certain amount of the cell mixture onto a plate.

The automatic cell dispensing device may further include a plate vision module.

The plate vision module may include a second lighting unit, a second optical system positioned to face the second lighting unit, a second camera, and a plate stage which is disposed between the second lighting unit and the second optical system and is two-dimensionally movable. The second camera may capture an image of the cell mixture on a transparent plate seated on the plate stage through the second optical system.

The number of cells contained in the cell mixture dispensed onto the plate may be calculated based on the image of the cell mixture on the plate captured by the second camera.

The automatic cell dispensing device may further include a z-axis transfer unit configured to adjust an autofocusing position by minutely moving the second optical system in a vertical direction, and autofocusing may be performed on a dome of the cell mixture dispensed on the plate in a z-axis direction for each of a plurality of layers to capture an image and perform image analysis for each layer.

An automatic cell dispensing method controlled by an information processing device or a control module and executed by the automatic cell dispensing device includes the following operations.

The automatic cell dispensing method may include: an operation of moving a pipette device to a position above a sample container and then lowering the pipette device to suction a cell mixture into a pipette tip; a cell measurement operation of capturing, by a tip vision module, an image of a certain area of the pipette tip one or more times in a state in which the pipette tip is positioned at a counting position and counting the number of cells in the pipette tip; and a dispensing operation of dispensing a certain volume of a mixture onto a plate.

The cell measurement operation may include: an operation of transferring the pipette tip, into which the cell mixture is suctioned, to the counting position; an imaging operation of capturing an image with a camera while stopping the pipette tip at the counting position; and an operation of analyzing the captured image and calculating the number of cells in a certain area of the pipette tip; wherein the imaging operation and the operation of the calculating are repeated, and when the number of cells reaches a certain number, the dispensing operation is performed.

The automatic cell dispensing device may further include a plate vision module configured to inspect the plate seated on a plate stage, and the automatic cell dispensing method may further include an operation of capturing, by the plate vision module, an image of the cell mixture dispensed onto the plate and inspecting the number of dispensed cells.

According to another aspect of the present invention, an automatic liquid handling system includes: a deck which is disposed at a lower side and on which one or more racks are mounted; a pipette device which is disposed on the deck and is three-dimensionally movable by an actuator and in which a pipette tip is coupled to a probe to suction and dispense a cell mixture; a tip vision module mounted on the deck to capture an image of the pipette tip containing the cell mixture suctioned from a sample container at a counting position; a cooling module which holds the sample container accommodating the cell mixture that contains cells or cell aggregates and cools and maintains the cell mixture; and a control module configured to calculate the number of the cells based on the captured image of the pipette tip into which the cell mixture is suctioned and control the pipette device to operate according to a pre-programmed method.

The automatic liquid handling system may further include a plate vision module configured to inspect a plate seated on a plate stage.

The plate vision module may include a second lighting unit, a second optical system positioned to face the second lighting unit, a second camera, and the plate stage which is disposed between the second lighting unit and the second optical system and is two-dimensionally movable in a horizontal (xy) direction.

The second camera may capture, through the second optical system, an image obtained by emitting light to pass through the cell mixture on the transparent plate seated on the plate stage from the second lighting unit, and the number of cells contained in the cell mixture dispensed onto the plate may be calculated based on the image of the cell mixture on the plate captured by the second camera.

A central axis of a holder of the cooling module, in which the sample container is inserted into and seated, may be coaxial with the counting position and positioned below the counting position.

The automatic liquid handling system may further include a z-axis transfer unit configured to adjust an autofocusing position by minutely moving the second optical system in a vertical direction, and autofocusing may be performed on a dome of the cell mixture dispensed on the plate in a z-axis direction for each of a plurality of layers to capture an image and perform image analysis for each layer.

The automatic liquid handling system may further include a gripper which is positioned on the deck and is three-dimensionally movable, and a heater seated in an area of the deck.

The gripper may transfer and seat a plate on the heater, and the heater heats the plate to a certain temperature to solidify the dispensed cell mixture.

According to still another aspect of the present invention, an automatic cell dispensing device includes a deck, pipette device which is movable along three axes by an actuator and in which a transparent pipette tip is coupled to a probe to suction and dispense a sample, and a tip vision module configured to capture an image of the pipette tip at a counting position.

After the cell mixture is suctioned into the pipette tip from a sample container by the pipette device, in a state in which the pipette tip is lifted to the counting position and stopped, the image of the pipette tip into which the cell mixture is suctioned may be captured by the tip vision module, and the number of cells in a certain area of a lower portion of the pipette tip may be calculated based on the captured image. When the number of the cells calculated reaches a certain number, the pipette device may move to dispense a certain amount of the cell mixture onto a plate.

The deck may include a fixed deck portion and a moving deck portion on which one or more components are detachably seated. The automatic cell dispensing device may further include a cooling module fitted into and seated in one section of the moving deck portion, and the cooling module may include a cooler block including a thermoelectric element and a cooling block seated on the cooler block and configured to insert and set a sample container from above.

After the cell mixture is suctioned into the pipette tip from the sample container by the pipette device, in a state in which the pipette tip is lifted to the counting position and stopped, imaging and counting may be repeated until the number of cells reaches a certain number.

### [Advantageous Effects]

According to one aspect of the present invention, in order to solve the problems of dilution-based dispensing, there is provided an automatic cell dispensing method and device for uniformly dispensing cells.

According to the present invention, there are provided an automatic liquid handling system having a modularized and compact configuration that is capable of uniformly dispensing cells and performing verification, and a processing method.

### [Description of Drawings]

FIG. 1 is a perspective view of an automatic liquid handling system according to one embodiment of the present invention.
FIG. 2 is a perspective view illustrating a state in which only a tip vision module, a plate vision module, and a cooling module are installed on a deck of the automatic liquid handling system according to one embodiment of the present invention.
FIG. 3 is a perspective view of the tip vision module according to one embodiment of the present invention.
FIG. 4 is a perspective view illustrating the tip vision module and a cooler block seated on the deck of the automatic liquid handling system according to one embodiment of the present invention.
FIG. 5 is a longitudinal cross-sectional view of a lighting module of the tip vision module and the cooling module seated on the deck of the automatic liquid handling system according to one embodiment of the present invention.

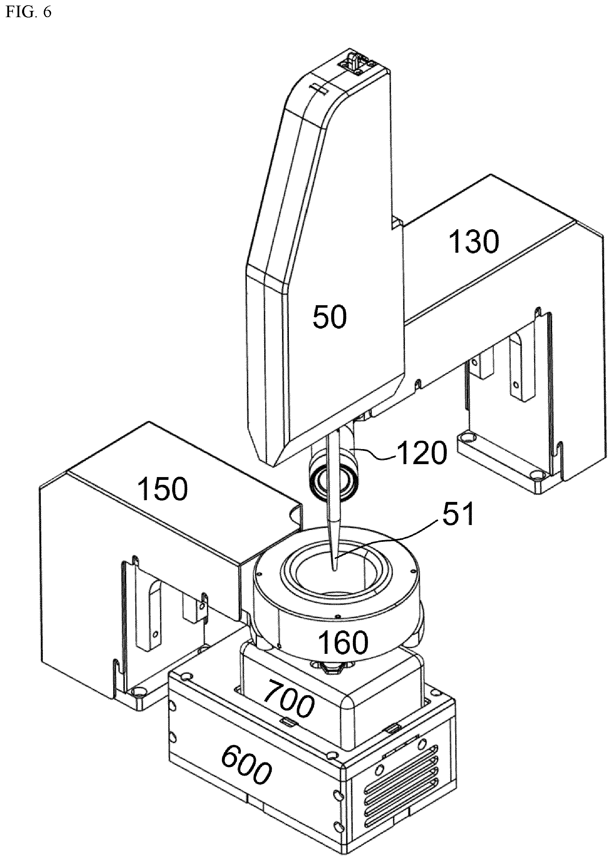

FIG. 6 is a perspective view illustrating relative positions of the tip vision module, the cooling module, and a pipette device according to one embodiment of the present invention and illustrating a state in which the pipette device is moved to a counting position to count cells after suctioning a cell mixture from a sample container seated on the cooling module.
FIG. 7 is a perspective view of a plate vision module and a plate stage of the automatic liquid handling system according to one embodiment of the present invention.
FIG. 8 is a perspective view illustrating the plate vision module of the automatic liquid handling system according to one embodiment of the present invention.
FIG. 9 is a perspective view illustrating the operation of the plate stage of the automatic liquid handling system according to one embodiment of the present invention.
FIG. 10 is an internal top view illustrating a state in which the tip vision module, the plate vision module, the cooling module, the plate stage, and all racks are installed on the deck of the automatic liquid handling system according to one embodiment of the present invention.
FIG. 11 is an image showing a preparation operation of a cell automatic processing method according to one embodiment of the present invention.
FIG. 12 shows images showing a cell mixture suction operation and a cell measurement operation of the automatic cell processing method according to one embodiment of the present invention.
FIG. 13 is an image captured in a cell measurement operation of the cell automatic processing method according to one embodiment of the present invention.
FIG. 14 is an image captured in a dispensed cell inspection operation.

### [Modes of the Invention]

Hereinafter, embodiments of the present invention will be described in detail with reference to the accompanying drawings.

An automatic liquid handling system is an experimental automation device designed to facilitate washing, dilution, incubation, dispensing, and moving of labware such that a large number of samples may be efficiently processed at high speed in new material development, new medicine development, and research.

From an operation of filling microplates used in biotechnology experiments or manufacturing to complex multi-stage pipetting operations such as plate reformatting, stamping, and serial dilution, tests requiring precision may be automated and repeatedly performed with minimal manpower, and errors in experimental results may be minimized.

An automatic liquid handling system according to one embodiment of the present invention may include various modules mounted on a deck and thus may be used not only for experiment automation but also for biomedicine production automation.

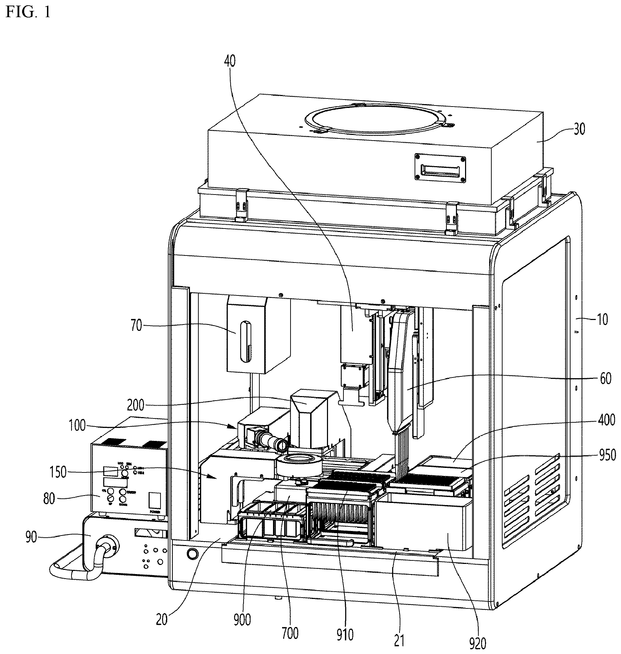

Hereinafter, a liquid handling system including an automatic cell dispensing device will be described in detail with reference to FIGS. 1 to 3.

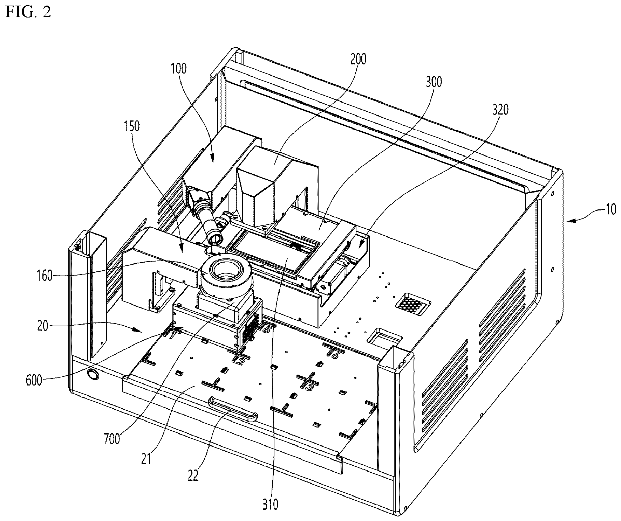

FIG. 1 is a perspective view of the automatic liquid handling system according to one embodiment of the present invention. FIG. 2 is a partial perspective view illustrating a state in which only a tip vision module, a plate vision module, and a cooling module are mounted on a deck of the automatic liquid handling system according to one embodiment of the present invention.

The automatic liquid handling system according to one embodiment of the present invention includes one or more transfer units, one or more pipette devices and a gripper that are three-dimensionally moved by the one or more transfer units, one or more vision modules, a case 10 that accommodates various components therein, and decks 20 and 21 which are disposed at a lower portion of the case and on which the one or more vision modules and one or more racks are mounted. Additionally, an opening and closing door (not shown) is formed in front of the case 10, and an air conditioning device 30 including a filter for maintaining a certain air cleanliness condition inside the case 10 is installed on an upper end of the case 10.

Referring to FIGS. 1 and 2, the transfer unit is disposed at an upper portion inside the case 10, and a first pipette device, a second pipette device, and the gripper coupled to the transfer unit may each be three-dimensionally moved inside the case by the transfer unit. The transfer unit may be a three-axis actuator, and the configurations of the transfer unit, the pipette device and the gripper may be provided as various types of a typical liquid handling system. For example, the transfer unit may include xyz three-axis actuators such as three y-axis transfer units each coupled to the same x-axis linear transfer unit, and a z-axis transfer unit coupled to each y-axis transfer unit, and a first pipette device 50, a second pipette device 60, and a gripper 40 may each be coupled to one z-axis transfer unit to independently move three-dimensionally in the case.

Each pipette device may be a general automatic pipette device including a stepper motor that supplies driving energy for suctioning/discharging a liquid, a pump operated by the stepper motor, a syringe, a pipette probe for seating a pipette tip, and the like. Through the operation of the pump and the syringe by the operation of the step motor driven by a separate control module, a liquid is suctioned and discharged into and from the pipette tip mounted on the pipette probe.

One or more pipette devices may be one-channel pipette devices in which one pipette tip is coupled to a single tip coupled to a probe, or multi-channel pipette devices including a multi-pipette probe such that a plurality of pipette tips are simultaneously coupled to a plurality of probes. In the present embodiment, the first pipette device 50 is a one-channel pipette device, and the second pipette device 60 is a multi-channel pipette device.

The specific configuration of the pipette device and the transfer unit is well known, and thus a detailed description thereof is omitted.

The deck is formed at a lower portion of the case such that one or more vision modules, the cooling module, a pipette tip array container, a sample plate, and the like are disposed thereon. Two side surfaces, a rear surface, and an upper surface of the deck are covered by a quadrangular case to form an internal space of a liquid handler.

Referring to FIG. 2, the deck includes a fixed deck portion 20 and a moving deck portion 21 that is slidable forward. The moving deck portion 21 disposed at a lower front portion of the case 10 includes a plurality of seating portions that are partitioned such that various modules including a cooling module 600 or racks such as a plate rack and a pipette tip rack may be mounted in each section, and a moving deck portion grip 22 for slidably moving the moving deck portion forward or backward. A rail or linear guide is formed at a lower portion of the moving deck portion such that the moving deck portion is slidable forward or backward.

The fixed deck portion 20 is fixedly disposed on both side surfaces and a rear surface of the moving deck portion 21, and a plurality of seating portions are formed such that two vision modules are mounted in left and rear areas of the fixed deck portion, and one or more racks are seated in a right area thereof.

Referring to FIG. 2, the tip vision module is mounted at a front one side of the fixed deck portion, and a plate vision module 200 is mounted at a rear one side of the fixed deck portion.

The tip vision module includes an imaging module 100 and a lighting module 150 and 160, captures an image of a cell mixture suctioned into a pipette tip from a sample container by a pipette device, and transmits the image to a control module 80 or an external information processing device. The control module 80 or the external information processing device includes a processor such as a central processing unit (CPU) or an application processor (AP), a memory, and a communication module, and executes a software program stored in the memory using a processor to control operations of components of the liquid handling system and processes image data received from the vision module to calculate the number of cells. Referring to FIG. 1, the control module may be disposed outside the case 10, but the present invention is not limited thereto. The control module may also be disposed inside the case.

The imaging module includes a lens unit including one or more lenses and a camera. The lighting module includes an annular lighting unit 160 having a hollow central portion, supports 150 and 152 supporting the annular lighting unit, a light source (not shown), and a light transmission unit (not shown) that transmits light from the light source to the annular lighting unit. The annular lighting unit is configured to radiate illumination light toward a central axis.

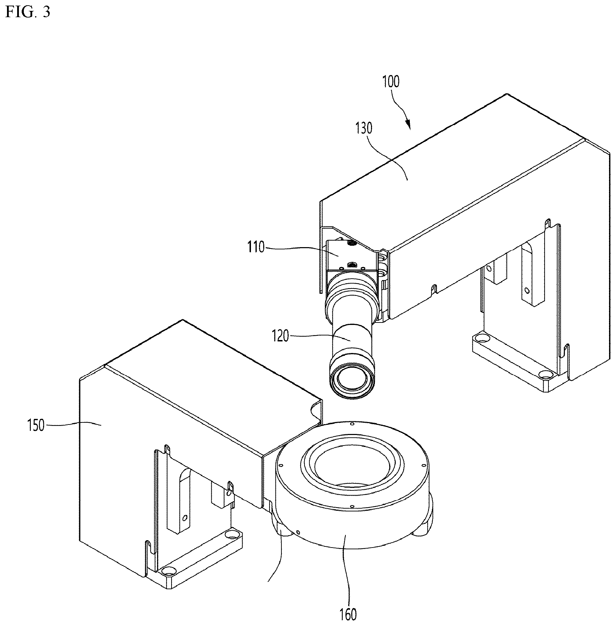

Referring to FIGS. 2 and 3, the support 150 supporting the annular lighting unit has a shape of an inverted "┐," a lower end portion thereof is fixed to a front left area of the fixed deck portion 20, and an upper end portion thereof horizontally extends to support the annular lighting unit 160 such that the annular lighting unit 160 is disposed above and spaced apart from a cooling module seating portion of the moving deck portion 21.

The imaging module 100 includes a ┐-shaped support 130, a camera 110 coupled to an upper front end portion of the ┐-shaped support 130, and a first lens unit 120 disposed at a front end portion of the camera 110. The camera 110 and the first lens unit 120 are positioned to focus on a counting position above the lighting unit.

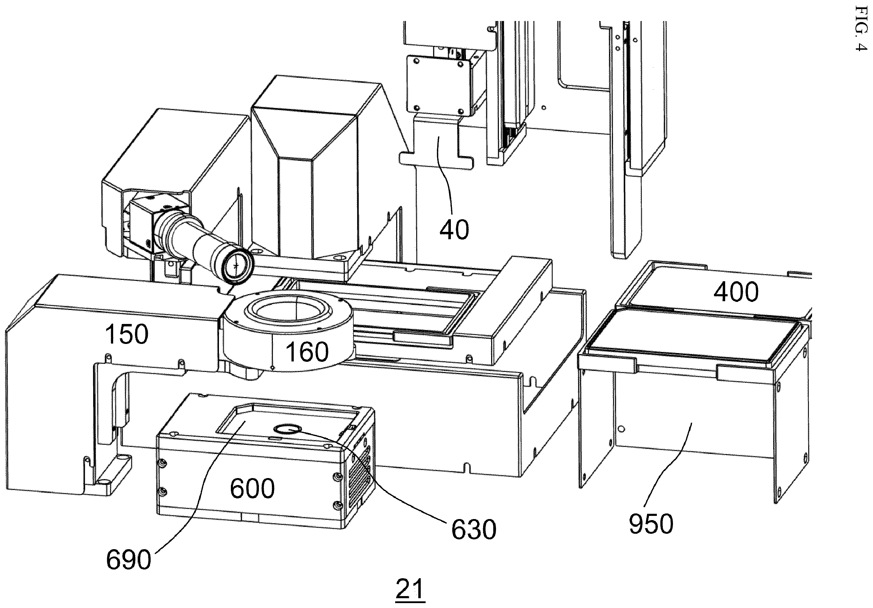

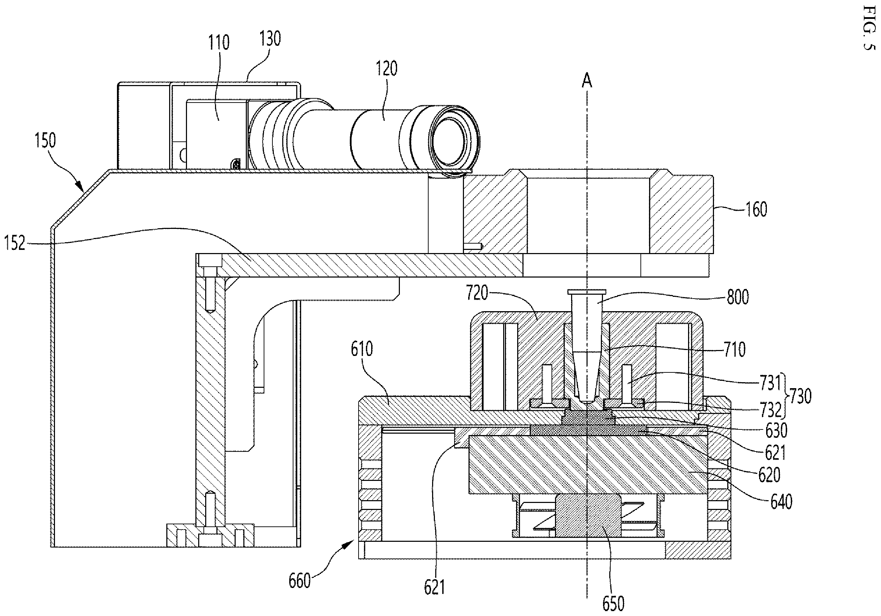

Meanwhile, FIG. 4 is a perspective view illustrating the tip vision module and a cooler block 600 seated on the deck of the automatic liquid handling system according to one embodiment of the present invention, in which a cooling block 700 is not seated. FIG. 5 is a longitudinal cross-sectional view of the lighting module of the tip vision module and the cooling module seated on the deck in the automatic liquid handling system according to one embodiment of the present invention.

The counting position is defined from an upper end of the annular lighting unit 160 to a certain height in a height direction (z) on a central axis A of the annular lighting unit. The certain height may correspond to a field of view of the camera 110, but the present invention is not limited thereto. The camera 110 captures an image of the pipette tip when the pipette tip is disposed at the counting position. That is, when the pipette tip is disposed at the counting position, a lower end of the pipette tip is disposed at the same level as the upper end of the annular lighting unit 160, and a central axis of the pipette tip is the same as the central axis A of the annular lighting unit.

The annular lighting unit 160 is disposed above and spaced apart from a first seating portion of the moving deck portion 21. Referring to FIGS. 2 to 5, the cooling module on which a sample container is seated is disposed on the first seating portion, and a z-direction central axis of the seated sample container is designed to be coaxial with the central axis A of the annular lighting unit. The moving deck portion may be slid forward to seat the sample container in a holder of a cooling module 600 and 700, and then the moving deck portion may be slid backward to allow the sample container to be coaxially disposed below the lighting unit along the central axis thereof.

Referring to FIGS. 4 and 5, the cooling module includes the cooler block 600 seated on the first seating portion of the moving deck portion 21 and the cooling block 700 mounted on a cooling block seating portion positioned at an upper end portion of the cooler block. It is preferable that the cooler block 600 and the cooling block 700 are provided separately to seat the cooling block 700 on the cooler block 600 seated on the moving deck portion 21. For experiments and the like, the moving deck portion 21 is slid forward to seat the cooling block 600 and then operate the cooling block 600. Meanwhile, in a state in which a sample container is mounted on the cooling block 700 that is separately refrigerated, the cooling block 700 is mounted on the cooling block 600, and then the moving deck portion 21 is slid backward to arrange the cooling block 700 such that the cooling block 700 is disposed below and spaced apart from the lighting unit.

The cooler block 600 includes a thermoelectric element 620, a heat sink 640 that is coupled to and in close contact with the thermoelectric element, one or more fans 650, a cooler case 660 with a shape of a quadrangular case that accommodates the thermoelectric element, the heat sink, and one or more fans therein, and a cooler cover 610 that covers the cooler case and has an upper surface on which a seating portion 690 for seating the cooling block is formed. A through-hole is formed in one area of the seating portion, and a heat transfer unit 630 is inserted into and fixed in the through-hole. The heat transfer unit 630 that is positioned to pass through one area of the seating portion is preferably a circular aluminum plate, but the present invention is not limited thereto. The heat transfer unit 630 may be made of another metal with excellent thermal conductivity such as copper or silver and may also have a shape of a quadrangular plate rather than a circular shape.

A heat absorption portion of the thermoelectric element 620 is coupled to and in close contact with a lower portion of the heat transfer unit 630 and absorbs heat from the heat transfer unit, and a heat generation portion is connected to the heat sink 640 at a lower side. One or more fans 650 positioned adjacent to a lower portion and side surfaces of the heat sink dissipate heat from the cooler block to the outside.

The cooler cover 610 is made of engineering plastic that is a material with excellent insulation properties such as poly-oxy-methylene (POM, acetal). The cooling block seating portion for seating the cooling block is concavely formed in one area of the upper end portion of the cooler cover 610. Alternatively, the seating portion may be provided in the form of a protruding edge to correspond to a shape of a lower edge of the cooling block such that the cooling block is positioned and fixed in a specific area of an upper surface of the cooler block.

The cooling block 700 includes a holder 710 for holding a sample container 800, a cooling case 720 that holds the holder therein and has a lower portion that is seated on the seating portion to allow the cooling block to be seated on the cooler block, and a fastening portion that fixedly couples the holder 710 to the cooling case.

The cooling case 720 is a case made of engineering plastic that is a material with excellent insulation properties such as POM (acetal). The cooling case 720 has a lower portion formed in a shape corresponding to a seating portion of the cooler block to be fitted into and seated on the seating portion, and a through-hole for fixing and holding the holder 710 having a U-shaped cross section is formed therein. For example, the seating portion of the cooler block is formed as a quadrangular concave portion with one corner that is cut off, and a lower portion of the cooling block 600 is provided in a corresponding shape (a quadrangular shape with one corner that is cut off) to be fitted into and seated on the seating portion of the cooler block.

The through-hole may have a cylindrical shape or a polygonal columnar shape, but the present invention is not limited thereto. Any shape that fits an outer periphery of the holder is sufficient.

Referring to FIG. 5, the through-hole for holding the holder has open upper and lower end portions and has a stepped portion at the upper end portion of the through-hole through which the cooling case protrudes by a certain thickness along the central axis A of the through-hole with the cylindrical shape. The stepped portion sets an upper limit when the holder is assembled, and at the same time, an upper end of the holder is not exposed to the outside, and thus an insulation operation is performed by the cooling case. An upper edge surface of the holder is blocked from the outside by the stepped portion, but upper portions of the holder and the through-hole are open to have a cross section with a certain size so that the sample container 800 may be inserted from above the cooling block 700 and seated. Preferably, an inner surface of the stepped portion at an upper side and an inner surface of an upper end portion of the holder at a lower side in contact therewith are coaxial with each other and have the same perimeter so that, in a state in which the cooling block 700 is assembled, an inner side surface of the stepped portion is smoothly and vertically connected to an inner side source of the holder in a line. That is, a protruding stepped portion of the cooling case is formed in surface contact with an upper edge of the holder 710 of which a cross section has a U shape.

The holder 710 with the U shape is made of a material with excellent thermal conductivity, has a cylindrical shape with a closed lower end, and has a space for holding a sample container therein, and a lower surface thereof is provided in surface contact with a heat transfer body of the cooler block. Accordingly, in a state in which the cooling block 700 is seated on the cooler block 600, the holder, the heat transfer body, and the thermoelectric element are sequentially and vertically disposed in a straight line to come into contact with each other so that a sample in the sample container seated in the holder is continuously maintained in a cooled state.

Meanwhile, a stepped portion is formed by cutting a lower outer edge of the holder 710 with the cylindrical shape with the closed lower portion, and a fastening portion is coupled to the stepped portion so that the holder is fixedly coupled to the cooling case. For assembly, a stepped portion for a fastening portion is formed at a lower edge of the holder such that the holder 710 may be inserted into the cooling case 720 from below and fastened and fixed. Referring to FIG. 5, an annular cutout portion is formed on the lower outer edge of the holder, an upper edge of the holder is defined by the stepped portion of the cooling case 720, and a lower portion is fixed to the cooling case by an annular fastening plate 732 fitted into the annular cutout portion and a screw 731.

Referring to FIGS. 4 and 5, the cooler block fitted into and seated on the deck and the cooling block fitted into and seated on the seating portion of the cooler block are disposed below the annular lighting unit 160, and the central axis A of the annular lighting unit 160 is disposed to be coaxial with the central axis A of the sample container which is fitted into and installed in the U-shaped holder of the cooler block. A lower surface of the U-shaped holder is in surface contact with the heat transfer unit of an upper surface of the cooler block, and the thermoelectric element and the heat sink are sequentially arranged at a lower portion.

The counting position is positioned on the central axis of the sample container, and the camera 110 is positioned to capture an image of the counting position.

Meanwhile, the cooler block 600 and the cooling block 700 may be integrally provided through a design change. In this case, the cooler case and the cooling case may be fixedly coupled by a method such as a bonding method, or two cases may be formed as one case through a design change. Other main components are the same as the above components, and thus redundant descriptions are omitted.

The liquid handling system according to one embodiment of the present invention may further include the plate vision module. Hereinafter, the plate vision module and a plate stage of the automatic liquid handling system according to one embodiment of the present invention will be described with reference to FIGS. 1, 2, 7, 8, and 9.

The plate vision module captures an image of a cell mixture dispensed onto a plate seated on the plate stage. Image data of a dome of the cell mixture dispensed onto the plate is transmitted to the control module or the external information processing device and analyzed to calculate the number of cells in the dome.

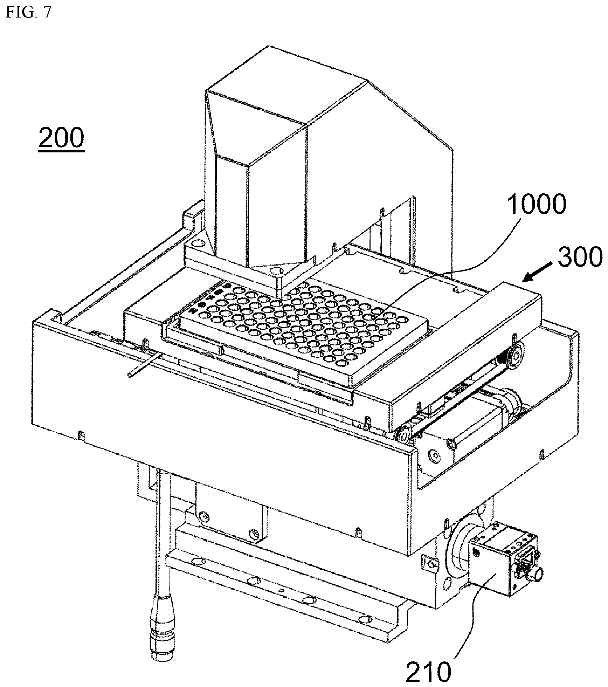

Referring to FIG. 2, the plate vision module 200 and a plate stage 300 are disposed on the fixed deck portion 20 at the rear of the cooling module. Referring to FIGS. 7 to 9, the plate vision module 200 includes an upper lighting, an optical system including a second lens unit disposed below the upper lighting to face the upper lighting, and a plate camera.

As shown in FIG. 7, the plate stage is disposed between an upper lighting 260 and the optical system thereunder. The plate stage 300 includes a plate seating portion 350 and a two-dimensional transfer unit 320 that moves the plate seating portion in a two-dimensional (x-axis and y-axis) horizontal direction. The upper lighting 260 is mounted on a stand 250 having a through-hole formed therein, and light from the upper lighting 260 is transmitted to an optical system 220 thereunder through the through-hole.

The plate seating portion 350 includes an internal through-hole formed to allow illumination light to pass through an individual well of a plate 1000, and a seating portion formed at an edge of the through-hole to physically support the plate 1000. The plate seating portion may be two-dimensionally moved by a two-dimensional transfer unit and may positioned below the upper lighting or may be moved to be away from the upper lighting through a slide transfer by the two-dimensional transfer unit. The plate seating portion 350 may be slidably moved to a position below the upper lighting for inspection. When the upper lighting is moved to the other side as much as possible from a position below the upper lighting, the plate that is seated on the plate seating portion may be away from the upper lighting to be completely exposed. This is to ensure that dispensing may be performed by the pipette device without hindrance in a state in which the plate is seated on the seating portion.

The plate stage includes a motor, a belt coupled to a rotational shaft of the motor, a linear motion guide, a cable chain, and a plate seating portion. Since there are various methods that can be easily implemented by ordinary technicians, a detailed description is omitted.

In order for a cell mixture suctioned into a pipette tip 51 from the sample container 800 seated on the cooling module 600 and 700 to be dispensed onto the plate 1000 seated on the plate stage 300, the two-dimensional transfer unit of the plate stage transfers the plate seating portion 350 to the right to prevent the upper lighting from interfering with the movement of the pipette device.

Meanwhile, a z-axis transfer unit 240 for transferring a second lens unit 220 in a vertical direction (z-axis) for autofocusing is connected to the second lens unit.

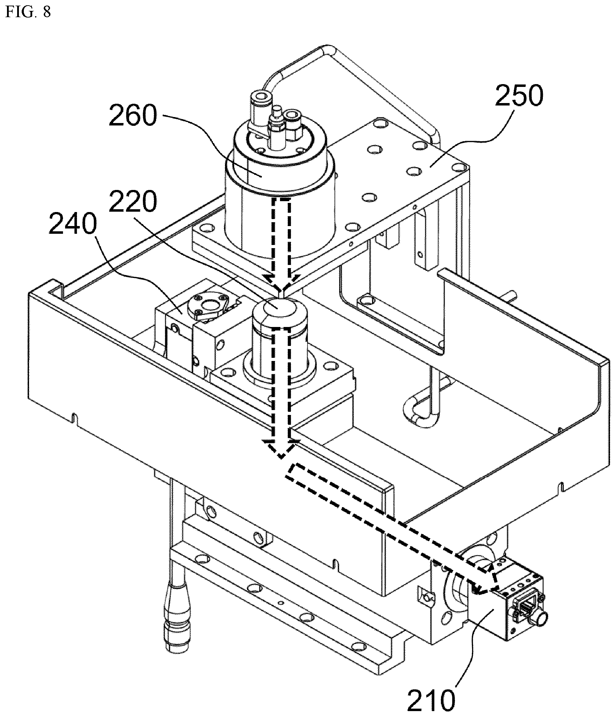

In order to efficiently use a space, a mirror is disposed at an inclined angle below the second lens unit, and the second camera 210 is horizontally disposed to capture image light focused by the second lens unit and reflected by the mirror. The dotted line in FIG. 8 schematically indicates an optical path.

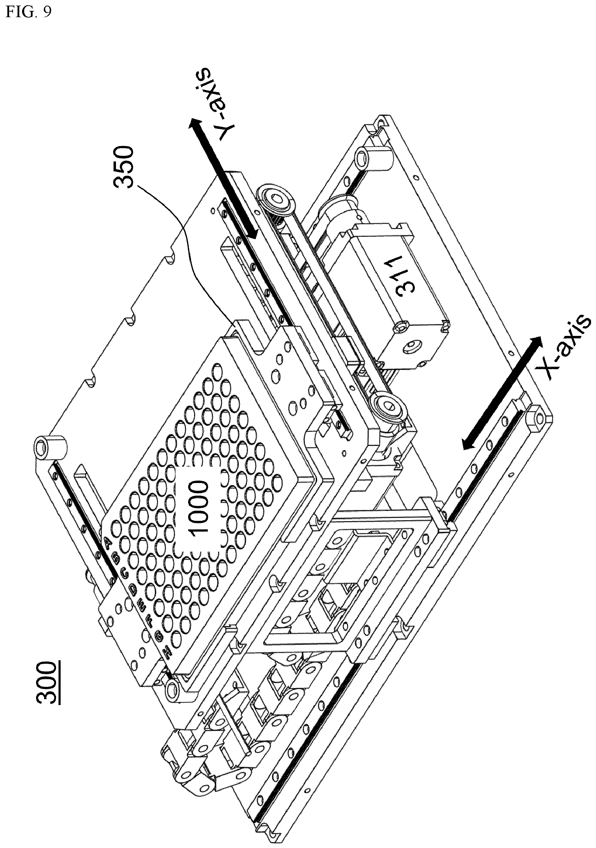

Referring to FIGS. 7 and 9, domes of a cell mixture dispensed onto the plate are each positioned in the well of the plate. In order to identify and check whether the number of cells measured by the tip vision module is correct, the plate vision module captures an image of the cell mixture dispensed onto the plate to measure the number of cells in the dome.

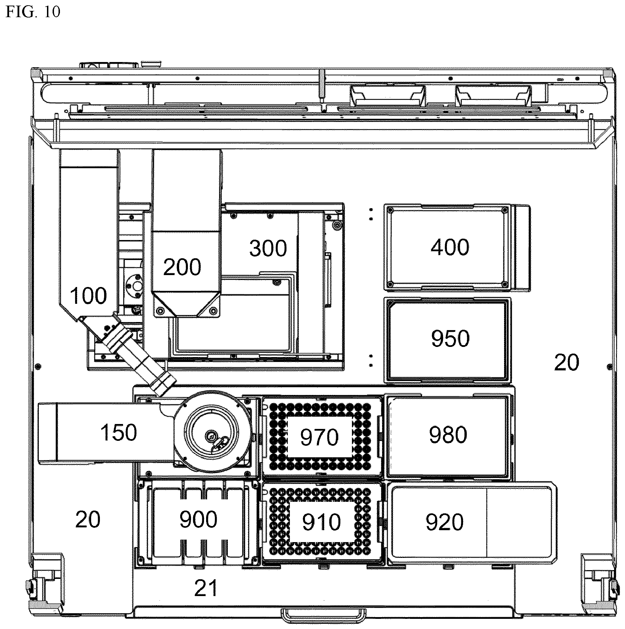

Hereinafter, a method of quantitatively dispensing and processing an organoid mixture using the automatic liquid handling system according to one embodiment of the present invention will be described in detail with reference to FIGS. 10 to 14.

### [Examples]

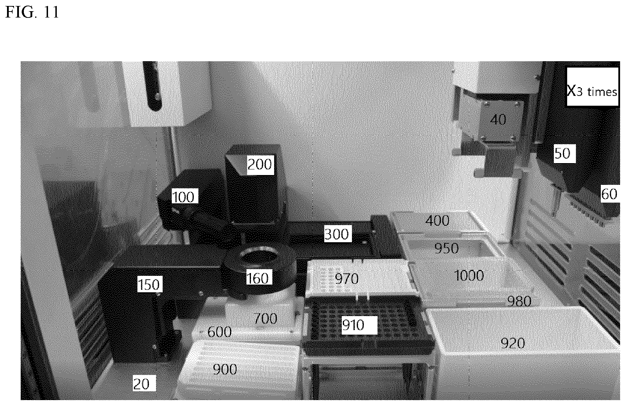

FIG. 10 is an internal top view illustrating a state in which the tip vision module, the plate vision module, the cooling module, the plate stage, and all racks are installed on the deck of the automatic liquid handling system according to one embodiment of the present invention. FIG. 11 is a perspective view of an image of the automatic liquid handling system of the top view of FIG. 10 captured in another direction.

Referring to FIGS. 10 and 11, the tip vision module and the plate vision module are mounted from a left front area to a rear area of the fixed deck portion 20 on the deck of the automatic liquid handling system. In addition, a heater 400, a cover rack 950 for mounting a cover, a first plate rack 980, and a waste container 920 are sequentially seated from the right rear to the front of the fixed deck portion 20.

On the moving deck portion 21, the cooler block 600, a first tip rack 970, a second tip rack 910, and a reservoir 900 for accommodating a medium are seated clockwise from the left rear.

The method of quantitatively dispensing and processing an organoid mixture using the liquid handling system according to the embodiment of the present invention includes all or some of the following operations. All or some of the following operations of the method may be automatically performed by a software program which is stored in the memory of the control module or the information processing device and executed by the processor.

### 1. Sample preparation operation (see FIG. 11)

A sample container (1.8 ml) containing 1.5 ml of a sample, a reservoir containing a medium, first and second tips, and a plate are prepared.

As the sample, a cell mixture in which an organoid and a matrigel are mixed is prepared in the sample container. Since the mixture of a cell and a matrigel easily solidifies at room temperature, the sample container is seated on the cooling module to maintain a cooled state during dispensing.

The cooling block 700 is stored separately in a refrigerator, and the moving deck portion 21 is slid forward to seat the cooling block 600 and then operate the the cooling block 600. Afterwards, after the sample container is seated on the cooling block 600 in a state in which the sample container is inserted into and seated in the holder 710 of the cold cooling block 700, when the moving deck portion 21 is slid backward, the cooling module is positioned below and spaced apart from the lighting unit. In this case, a cooling temperature is preferably about 4°C, and in a state in which the cooling block 700 is seated on the cooler block 600, the moving deck portion 21 is slid backward, a central axis of the sample container is disposed to coincide with the central axis A of the lighting unit.

A z-direction central axis of the seated sample container is designed to be disposed to be coaxial with the central axis A of the annular lighting unit. The moving deck portion may be slid forward to seat the sample container in the holder of the cooling module 600 and 700, and then the moving deck portion may be slid backward to arrange the sample container below the lighting unit to be coaxial with the central axis of the lighting unit.

Since the cooler block 600 is operated and cooled to a temperature of 4°C, and the sample container is held in the cooling block 700 cooled to a temperature of 4°C in advance, the cell mixture inside the sample container is maintained in a cooled state during a dispensing operation by the heat transfer body and the thermoelectric element of the cooler block 600 in surface contact with the holder of the cooling block 700 and thus is not solidified.

Meanwhile, a medium is put into the reservoir, a first tip (50 µl pipette tip) array is seated on the first tip rack 970, a second tip (200 to1,000 µl pipette tip) array is seated on the second tip rack 910, and the plate covered with a cover is seated on the first plate rack 980.

The sample preparation operation may be automatically performed, but may also be manually performed.

### 2. Plate moving operation in which the gripper transfers the plate on the plate rack 980 to the plate stage 300 and then removes the cover of the plate and moves the plate to the cover rack 950

The gripper 40 transfers the plate 1000 covered with the cover to the plate stage 300 and seats the plate 1000 on the plate seating portion 350, and then picks up and transfers only the cover and places the cover on the cover rack 950. In this case, the plate mounted on the seating portion of the plate stage 300 is positioned in a right area away from a lower area of the plate vision module 200 such that sample dispensing is possible using the pipette device.

According to the need of a user, an operation in which the gripper moves the plate to the plate stage may be omitted or may be performed after dispensing on the plate. For example, after a dispensing operation is performed by removing only the cover in a state in which the plate is placed on the plate rack 980, an inspection operation, which will be described below, using the plate vision module may be performed, or after dispensing is completed, the plate in which dispensing is performed may be transferred to the plate stage to perform an inspection. Other variations are possible.

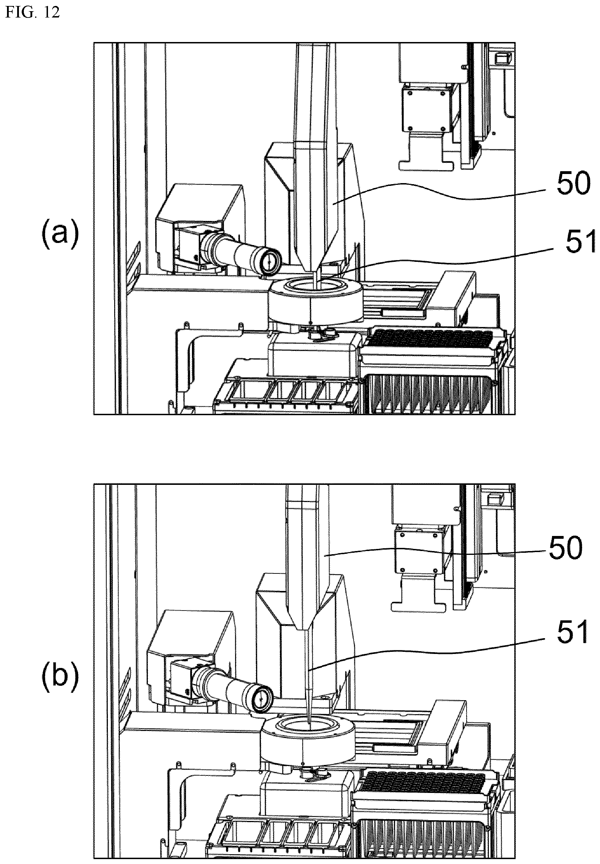

### 3. Sample (mixture of cell and matrigel) suction operation (see FIG. 12A)

The one-channel pipette device moves to a position above the sample container 800 and the annular lighting unit 160 and then is lowered along the central axis A of the annular lighting unit to suction 50 µl of the cell mixture into the pipette tip. In this case, the one-channel pipette device suctions the cell mixture from the sample container seated on the cooling module.

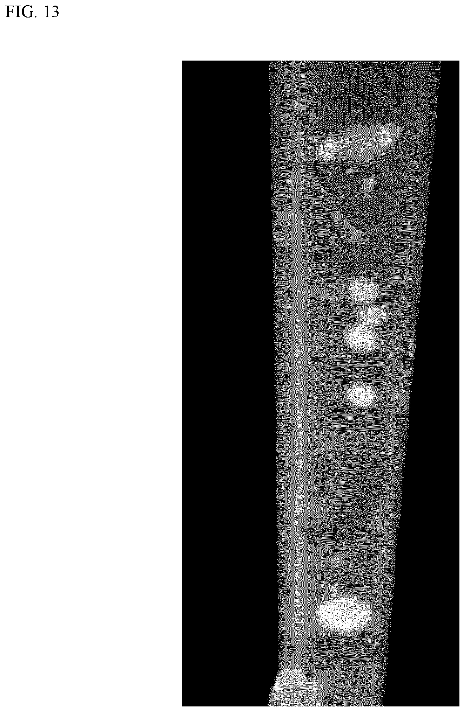

### 4. Cell measurement operation (see FIGS. 12B and 13)

A cell measurement operation, in which the tip vision module captures an image of the suctioned cell mixture and counts the number of cells in the pipette tip, includes the following three operations.

### Operation 4-1 of transferring the pipette tip, in which a sample mixture is suctioned by the pipette device, upward to move the pipette tip to a counting position just above the lighting unit

The one-channel pipette device 50 moves the pipette tip to the counting position at an upper end portion of the annular lighting unit 160 along the central axis A of the annular lighting unit. Here, the counting position is positioned on the central axis A of the lighting unit and is a position at which a lower end of the pipette tip is just away from the annular lighting unit 160. Specifically, the counting position is a position at which the pipette tip is arranged on the central axis A of the lighting unit, and the lower end of the pipette tip is at the same level as an upper end of the annular lighting unit 160.

### Imaging operation 4-2 of, when the pipette tip is positioned at the counting position, stopping the pipette, and then capturing an image with the camera

When the one-channel pipette device 50 is stopped in a state in which the pipette tip is positioned at the counting position, the imaging module 100 captures an image of the transparent pipette tip containing the cell mixture to obtain an image of the cell mixture contained in the tip. However, the camera 110 and the lens unit 120 are disposed to correspond to the counting position, and a certain height at which an image of the tip is captured is also determined in advance.

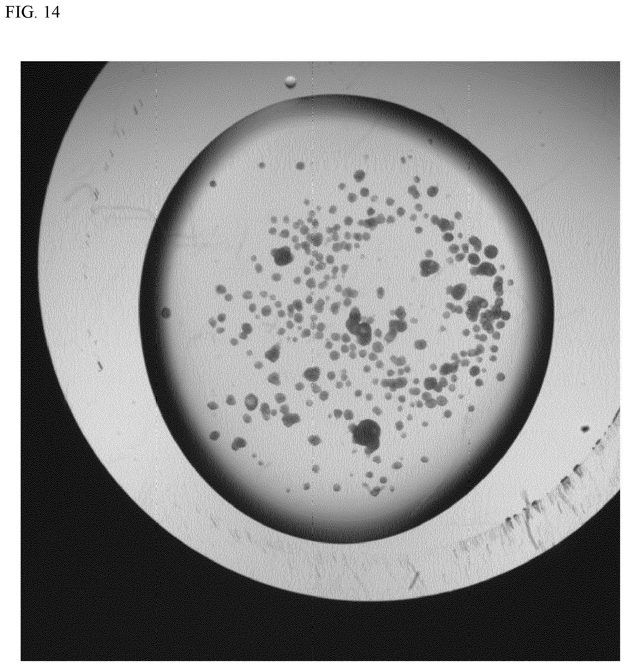

FIG. 14 shows an example of an image captured during the cell measurement operation. The image is captured at a certain height below the lower end of the pipette tip. A plurality of organoids can be confirmed inside a suspension.

### Operation 4-3 for calculating the number of cells included in a certain area in the cell mixture by analyzing the captured image

The number of cells inside a certain height (volume) from the lower end of the tip is calculated based on the height (volume), and the imaging and calculation operations are repeated until a preset number of cells are counted. A specific method of calculating the number of cells may be performed by extracting areas corresponding to cells from an image through image analysis in the control module or a separate external information processing device and calculating the number of such areas. Such image analysis is possible using common methods.

An image is captured and a calculation is performed at a certain sampling time interval, and when a certain number of organoids enter a certain area, a corresponding volume is dispensed by the certain area. For example, when 30 organoids are counted in a 5 µl area of the tip, 5 µl is dispensed. A dispensing process is described in detail below.

### 5. Dispensing operation of dispensing a certain volume of a mixture onto the plate

When the preset number of cells is counted in the cell measurement operation, the pipette device moves to a position above the plate and dispenses a certain volume of the cell mixture into the well of the plate. In this case, the certain volume corresponds to a height of the tip determined based on counting in operation 4-3. Specifically, the certain volume may be a volume of a mixture corresponding to the height of the tip determined based on the counting but may also be a volume smaller than the volume of the mixture by a certain amount. That is, when the preset number of organoids is measured, a mixture of, for example, 5 µl to 50 µl of the mixture is dispensed onto the plate. Suctioning, measuring, and dispensing processes may be performed on the wells to uniformly dispense the mixture into a plurality of wells of the plate.

### 6. Inspection operation of dispensed cell

In order to check whether an organoid mixture dispensed onto the plate is well dispensed, a dome formed in each well is inspected using the plate vision module.

When the sample is dispensed using the pipette device in a state in which the plate is seated on the plate seating portion 350, the plate is positioned below the lighting 260 through slide transfer of the two-dimensional transfer unit 320, and then sequential inspection is performed on each dome by the plate vision module 200. While the two-dimensional transfer unit 320 moves the plate seating portion such that the domes of the cell mixture dispensed below the lighting 260 are sequentially positioned, the two-dimensional transfer unit 320 allows light from the lighting 260 to pass through the plate and the dome to be transmitted to the second optical system thereunder.

FIG. 14 is an example of an image of the dome, which is dispensed onto the plate, captured by the second camera 210.

The plate seated on the seating portion 350 by the two-dimensional transfer unit 320 is moved horizontally to sequentially capture images of individual domes on the plate using the camera of the plate vision module and perform dome image analysis. In this case, since the dome has a thickness, in order to confirm the exact number of cells included in each dome, the second lens unit 220 is automatically and minutely moved by a second lens unit transfer unit 240 for each layer in one dome to perform autofocusing and capture an image and perform image analysis for each layer. The total number of cells in the dome is calculated by adding the number of cells (organoids) per layer for each dome through image analysis by an information processing device.

Dome (well) information in which the number of cells that is greater or less than or equal to a set certain cell number is detected may be confirmed by the control module or the information processing device.

### 7. Plate covering operation

The gripper picks up the cover of the plate and closes the plate with the cover again to prevent contamination.

### 8. Heating operation

The plate is picked up with the gripper, transferred, mounted on the heater 400, and heated at a temperature of 37°C for 15 minutes to solidify a dispensed solution. The heater is preferably an electric type.

### 9. Medium supply operation

After the plate is transferred from the heater 400 to the plate rack using the gripper, the cover of the plate is opened to move the covert to a rack, and 200 µl of a medium is dispensed onto a dome, in which a medium is prepared, using an 8-channel pipette.

10. The gripper picks up the cover of the plate and closes the plate with the cover again.

Through the above device and method, while the movement of the pipette device is minimized, uniform cell dispensing and incubating can be automatically performed with improved efficiency and precision in a short period of time.

### [Comparative Example]

As Comparative Example, a conventional dilution-based manual dispensing process will be described.
1. A sample container containing a mixture of a cell (organoid) and a matrigel is prepared at a temperature of 4°C.
   Since a mixture of a cell and a matrigel begins to solidify at room temperature, the sample container (vial tube) containing the mixture is put into a cooled block, or a tube is put into ice.
2. 5 µl to 10 µl of the mixture of the cell and the matrigel is suctioned into a pipette tip and dispensed to a plate to form a dome. In this case, the dispensing is dispensing based on dilution and is performed by assuming that the same number of cells is contained in the same mixture without counting of the number of cells, and thus the number of cells contained in the dome formed on the plate is not uniform for each dome.
3. The dome of the mixture is incubated at a temperature of 37°C for 10 minutes.
4. 200 µl of media are added to the dome.

### [Industrial Applicability]

An automatic cell dispensing device, a liquid handling system including the same, and an automatic cell dispensing method according to the present invention can be used in fields such as the manufacturing of biological experiment equipment including three-dimensional cell culture and biopharmaceutical manufacturing industry including mass cell culture.

## Claims

1. An automatic cell dispensing device for uniformly and automatically dispensing a cell mixture, the automatic cell dispensing device comprising:
a pipette device which is movable along three axes by an actuator and in which a pipette tip is coupled to a probe to suction and dispense a cell mixture; and
a tip vision module configured to capture an image of the pipette tip in which the cell mixture is suctioned from a sample container by the pipette device at a counting position;
wherein the number of cells is calculated based on a captured image of the cell mixture suctioned into the pipette tip.

2. The automatic cell dispensing device of claim 1, wherein the tip vision module includes an imaging module and a lighting module,
the imaging module includes a first optical system including one or more lenses and a first camera,
the lighting module includes a first lighting unit having an annular shape,
the first optical system and the first camera are disposed such that the first camera captures an image of the counting position at an annular center of an upper end portion of the first lighting unit,
the sample container is seated to be coaxially positioned below the first lighting unit along an annular central axis thereof, and
in a state in which the cell mixture from the sample container is suctioned into the pipette tip, the pipette device is vertically lifted and moved to the counting position.

3. The automatic cell dispensing device of claim 1 or 2, wherein a cooling module on which the sample container is seated is disposed below the counting position, and
the cooling module includes a thermoelectric element, a heat sink coupled to and in close contact with a lower portion of the thermoelectric element, one or more fans, a holder positioned on the thermoelectric element, and a cooler case,
wherein the holder has an upper end that is open such that the sample container is inserted from above and seated, and a lower end that is directly coupled to and in close contact with an upper end of the thermoelectric element or coupled through a heat transfer unit, and
the cooler case accommodates the thermoelectric element, the heat sink, and the one or more fans therein.

4. The automatic cell dispensing device of claim 1 or 2, wherein a cooling module on which the sample container is seated is disposed below the counting position, and
when a certain amount of a sample is suctioned into the pipette tip from the sample container by the pipette device and then is moved upward and positioned at the counting position, an image of a certain area of a lower portion of the pipette tip is captured from a side by the tip vision module,
the automatic cell dispensing device further includes an information processing device configured to analyze the captured image to calculate the number of cells in a certain area of the pipette tip,
a process of capturing an image and calculating the number of cells through image analysis is repeated one or more times until the calculated number of cells reaches a certain number,
when the calculated number of cells reaches the certain number, the pipette device dispenses a certain amount of the cell mixture onto a plate, and
the information processing device includes a processor, a memory, and a communication module and is a part of the automatic cell dispensing device or a separate external device.

5. The automatic cell dispensing device of any one of claims 1 to 4, further comprising a plate vision module,
wherein the plate vision module includes a second lighting unit, a second optical system positioned to face the second lighting unit, a second camera, and a plate stage which is disposed between the second lighting unit and the second optical system and is two-dimensionally movable,
the second camera captures an image of the cell mixture on a transparent plate seated on the plate stage through the second optical system, and
the number of cells contained in the cell mixture dispensed onto the plate is calculated based on the image of the cell mixture on the plate captured by the second camera.

6. The automatic cell dispensing device of claim 5, further comprising a z-axis transfer unit configured to adjust an autofocusing position by minutely moving the second optical system in a vertical direction,
wherein autofocusing is performed on a dome of the cell mixture dispensed on the plate in a z-axis direction for each of a plurality of layers to capture an image and perform image analysis for each layer.

7. An automatic cell dispensing method that is performed by the automatic cell dispensing device of claim 1, the automatic cell dispensing method comprising:
an operation of moving a pipette device to a position above a sample container and then lowering the pipette device to suction a cell mixture into a pipette tip;
a cell measurement operation of capturing, by a tip vision module, an image of a certain area of the pipette tip one or more times in a state in which the pipette tip is positioned at a counting position and counting the number of cells in the pipette tip; and
a dispensing operation of dispensing a certain volume of a mixture onto a plate.

8. The automatic cell dispensing method of claim 7, wherein the cell measurement operation includes:
an operation of transferring the pipette tip, into which the cell mixture is suctioned, to the counting position;
an imaging operation of capturing an image with a camera while stopping the pipette tip at the counting position; and
an operation of analyzing the captured image and calculating the number of cells in a certain area of the pipette tip;
wherein the imaging operation and the operation of the calculating are repeated, and when the number of cells reaches a certain number, the dispensing operation is performed.

9. The automatic cell dispensing method of claim 7 or 8, wherein the automatic cell dispensing device further includes a plate vision module configured to inspect the plate seated on a plate stage, and
the automatic cell dispensing method further includes an operation of capturing, by the plate vision module, an image of the cell mixture dispensed onto the plate and inspecting the number of dispensed cells.

10. An automatic liquid handling system comprising:
a deck which is disposed at a lower side and on which one or more racks are mounted;
a pipette device which is disposed on the deck and is three-dimensionally movable by an actuator and in which a pipette tip is coupled to a probe to suction and dispense a cell mixture;
a tip vision module mounted on the deck to capture an image of the pipette tip containing the cell mixture suctioned from a sample container at a counting position;
a cooling module which holds the sample container accommodating the cell mixture that contains cells or cell aggregates and cools and maintains the cell mixture; and
a control module configured to calculate the number of the cells based on the captured image of the pipette tip into which the cell mixture is suctioned and control the pipette device to operate according to a pre-programmed method.

11. The automatic liquid handling system of claim 10, further comprising a plate vision module configured to inspect a plate seated on a plate stage;
wherein the plate vision module includes a second lighting unit, a second optical system positioned to face the second lighting unit, a second camera, and the plate stage which is disposed between the second lighting unit and the second optical system and is two-dimensionally movable in a horizontal (xy) direction,
the second camera captures, through the second optical system, an image obtained by emitting light to pass through the cell mixture on the transparent plate seated on the plate stage from the second lighting unit,
the number of cells contained in the cell mixture dispensed onto the plate is calculated based on the image of the cell mixture on the plate captured by the second camera, and
a central axis of a holder of the cooling module, in which the sample container is inserted into and seated, is coaxial with the counting position and positioned below the counting position.

12. The automatic liquid handling system of claim 11, further comprising a z-axis transfer unit configured to adjust an autofocusing position by minutely moving the second optical system in a vertical direction,
wherein autofocusing is performed on a dome of the cell mixture dispensed on the plate in a z-axis direction for each of a plurality of layers to capture an image and perform image analysis for each layer.

13. The automatic liquid handling system of any one of claims 10 to 12, further comprising:
a gripper which is positioned on the deck and is three-dimensionally movable; and
a heater seated in an area of the deck;
wherein the gripper transfers and seats a plate on the heater, and the heater heats the plate to a certain temperature to solidify the dispensed cell mixture.

14. An automatic cell dispensing device comprising:
a deck;
a pipette device which is movable along three axes by an actuator and in which a transparent pipette tip is coupled to a probe to suction and dispense a sample; and
a tip vision module configured to capture an image of the pipette tip at a counting position;
wherein, after the cell mixture is suctioned into the pipette tip from a sample container by the pipette device, in a state in which the pipette tip is lifted to the counting position and stopped, the image of the pipette tip into which the cell mixture is suctioned is captured by the tip vision module, and
the number of cells in a certain area of a lower portion of the pipette tip is calculated based on the captured image,
wherein, when the number of the cells calculated reaches a certain number, the pipette device moves to dispense a certain amount of the cell mixture onto a plate.

15. The automatic cell dispensing device of claim 14, wherein the deck includes a fixed deck portion and a moving deck portion on which one or more components are detachably seated and which is movable,
the automatic cell dispensing device further includes a cooling module fitted into and seated in one section of the moving deck portion,
the cooling module includes a cooler block including a thermoelectric element and a cooling block seated on the cooler block and configured to insert and set a sample container from above, and
after the cell mixture is suctioned into the pipette tip from the sample container by the pipette device, in a state in which the pipette tip is lifted to the counting position and stopped, imaging and counting are repeated until the number of cells reaches a certain number.
